# EUROPEAN PATENT APPLICATION

(11) **EP 4 293 008 A1**
(43) Date of publication of application: **20.12.2023**
(21) Application number: 23178619.5
(22) Date of filing: 12.06.2023
(51) Int. Cl.: C07C 51/09, C07C 59/08, C07C 59/06

(54) **METHOD FOR THE DEPOLYMERIZATION OF A POLY(ALPHA-HYDROXY ACID)**

(30) Priority: 13.06.2022 LU 502257
(71) Applicant: Universität Hamburg, 20148 Hamburg (DE)
(72) Inventor: Enthaler, Stephan, 20146 Hamburg (DE)
(74) Representative: Stüven, Ralf

(57) **Abstract**

The invention relates to a method for the depolymerization of a poly(α-hydroxy acid), for example a poly(lactic acid). The method comprises the steps of:
a) contacting the poly(α-hydroxy acid) with water; and
b) heating the mixture of the poly(α-hydroxy acid) and water in the presence of a catalyst suitable for catalyzing the depolymerization of the poly(α-hydroxy acid).

## Description

The invention relates to a method for the depolymerization of a poly(α-hydroxy acid).

Plastics obtained from renewable raw materials can be valuable for a future circular and resource-efficient chemical industry and economy. In addition, these plastics have some advantages over plastics made from fossil raw materials and are becoming increasingly important. Biobased and biodegradable poly(α-hydroxy acid) plastics like, for example, poly(lactide), PLA, or poly(glycolide), PGA, are therefore widely used today in order to replace petrochemical polymers in, for example, packaging applications (see, e.g., Jem K.J., Tan B., The development and challenges of poly(lactic acid) and poly(glycolic acid), Advanced Industrial and Engineering Polymer Research, Volume 3, Issue 2, 2020, Pages 60-70, doi.org/10.1016/j.aiepr.2020.01.002).

Recently, poly(lactide) plastics (PLA) have established themselves as most important representatives of biodegradable bioplastics, with an annual worldwide production volume of approx. 0.3 million tons (2020). PLA plastics are accessible through a multi-stage process based on renewable raw materials. In biological conversion, carbon dioxide, water and solar energy are first converted into biomass, which is then converted into lactic acid. The lactic acid then undergoes polycondensation to produce the PLA polymer/oligomer. Another option is the oligomerization of lactic acid and subsequent degradation to lactide, the dimeric ester of lactic acid, which can undergo ring-opening polymerization to form PLA with significantly longer chain lengths. PLA plastics have a wide range of applications, but mainly in the temporary food packaging sector. After the PLA plastic has served its purpose, it is treated as waste as so-called End-of-Life-PLA (EoL-PLA).

In contrast to many petroleum-based plastics, biodegradability is being discussed, but the common residence time in composting plants is too short for complete degradation. Therefore, EoL-PLA is incinerated to release the stored energy and produce CO₂ and water that can go through the processes again, i.e., be used for the production of PLA. In contrast to plastics based on fossil raw materials, a circular process and almost CO₂ neutrality can be achieved in a short time. However, a disadvantage of this approach is the need to replace the EoL-PLA with fresh PLA, which requires land use and cultivation time, which can lead to competition with other agricultural commodities. To overcome these issues/limitations, recycling of EoL-PLA plastics can be a useful tool. However, no industrial recycling methods for EoL-PLA have been established to date. In this regard, chemical recycling based on depolymerization and polymerization can offer advantages. Specifically, in a primary depolymerization step, the EoL-PLA is converted into suitable monomers that can be used in the second part, polymerization, to generate new PLA. Consequently, the chemical functionalities are embedded in a cycle. It is worth mentioning that the monomers obtained in the depolymerization are also the monomers used in the industrially established polymerization processes, so that an introduction of the depolymerization products into the established polymerization processes is possible. In contrast to other established recycling methods, such as mechanical recycling and downcycling, the properties of the chemically recycled polymer are not linked to the quality of the End-of-Life plastic, i.e., the quality can be adapted to requirements in each cycle. In the case of chemical recycling of EoL-PLA, for example, there are several prior art approaches for depolymerization (e.g., hydrolysis, glycolysis, methanolysis). Hydrolysis is considered to be advantageous, since lactic acid is obtained as a depolymerization product, which serves as the starting material for industrial PLA syntheses (see, for example, US 5229528 A; EP 0244114 A1; Teixeira, S., Eblagon, K.M., Miranda, F., R. Pereira, M.F., Figueiredo, J.L. Towards Controlled Degradation of Poly(lactic) Acid in Technical Applications. C 2021, 7, 42. doi.org/10.3390/c7020042). However, in the prior art methods using hydrolysis of poly(lactic acid), long reaction times (1-3 h, for example) are required (see, e.g., US 5229528 A), or PLA oligomers or short-chain PLAs are mainly obtained (EP 0244114 A1). Further, stoichiometric amounts of additional reactants or solvents are required.

Alberti and Enthaler 2020 (Alberti, C., Enthaler, S. Depolymerization of End-of-Life Poly(lactide) to Lactide via Zinc-Catalysis, Chemistry Select 2020, 5, 14759, doi.org/10.1002/slct.202003979) describe the depolymerization of End-of-Life poly(lactide) to lactide via zinc-catalysis by reactive distillation.

Cheung *et al.* 2020 (Cheung, E., Alberti, C., Enthaler, S. Chemical Recycling of End-of-Life Poly(lactide) via Zinc-Catalyzed Depolymerization and Polymerization, ChemistryOpen 2020, 9, 1224, doi.org/10.1002/open.202000243) describe the chemical recycling of End-of-Life poly(lactide) via Zinc-catalyzed depolymerization and polymerization, using methanol as depolymerization reagent.

US 2013/0096342 discloses a method for the depolymerization of polyesters such as post-consumer polylactic acid in an alcoholic solution containing methanol or ethanol and depolymerization catalysts, e.g., an alkali metal hydroxide or alkali metal carbonate, optionally in conjunction with ultrasonic treatment.

There is still a need for methods that make recycling of poly(α-hydroxy acid) plastics, in particular PLA, more efficient. It is an object of the invention to provide such a method.

In a first aspect the invention provides a method for the depolymerization of a poly(α-hydroxy acid), the method comprising the steps of.
a) contacting the poly(α-hydroxy acid) with water; and
b) heating the mixture of the poly(α-hydroxy acid) and water in the presence of a catalyst suitable for catalyzing the hydrolytic depolymerization of the poly(α-hydroxy acid).

The method of the invention provides a method with the help of which poly(α-hydroxy acid) plastics (PHA plastics), for example poly(lactide) plastics, PLA, can efficiently be depolymerized, for example to enable a chemical recycling of the polymer. It has surprisingly been found that PHA plastics like PLA, for example, can efficiently be hydrolyzed in the presence of a suitable catalyst. In contrast to known chemical depolymerization methods, the catalyzed hydrolytic depolymerization according to the invention does not require any additional solvents. Rather, water is used as the sole reactant and solvent. The method of the invention involves comparatively short reaction times, i.e., reaction times ≤ 60 min, or even ≤ 45. Further, PHA waste (End-of-Life) plastics can be used in the method of the invention, which otherwise would be incinerated and thus withdrawn from the technical cycle. Low-quality End-of-Life PHA plastics can be decomposed to the monomers from which they are composed, which in turn can be used to produce high-quality PLA again. The quality of newly produced PHA from the products of the catalyzed hydrolytic depolymerization method of the invention is independent from the quality of the End-of-Life PHA used as starting material. In addition, the method of the invention has a high tolerance towards contaminants (e.g., dyes, adhesives, biological contaminants, paper) and towards the presence of other plastics and can thus also be used with impure, e.g., unsorted, incorrectly or badly sorted, plastic fractions. PHA plastics can selectively be converted to their respective monomers, PLA to lactic acid, for example, with the method of the invention, enabling chemical sorting/separation in case of plastic mixtures. Other advantages of the method of the invention are an increased space-time yield and the possibility to use lower reaction temperatures due to the use of suitable catalysts, e.g., the robust and active catalyst zinc(II) acetate. Consequently, the method of the invention allows for an efficient recycling of PHA plastics without the need for using problematic, e.g., toxic, and/or expensive solvents like methanol. This makes it possible to increase recycling rates to achieve sustainability goals.

In the method of the invention, the poly(α-hydroxy acid), PHA, is converted to the monomers that make up the poly(α-hydroxy acid), for example, lactic acid in case of PLA, in the presence of a suitable catalyst using water as the sole depolymerization reagent and solvent. The resulting monomers are the industrially relevant starting materials for the reverse reaction (polymerization/polycondensation) to synthesize new PHA. Furthermore, the water used is released again in the polymerization reaction and can be used for further depolymerization steps, so that no waste products are produced during the chemical recycling process.

The terms "poly(α-hydroxy acid)", PHA, or "poly(α-hydroxy acid) plastic" are used herein for a chemical compound of the chemical formula (-O-CR¹R²-CO-)ₙ, i.e, a polymer or copolymer having a single type of an alpha hydroxy acid ester or a mixture of two or more different types of alpha hydroxy acid esters as repeating units, as shown in the following general structure 1

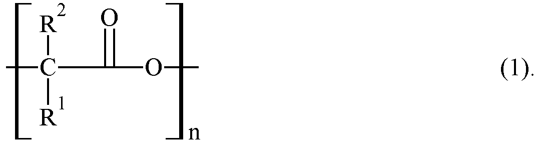

R¹ and R² can, independently from each other and for each occurrence in the polymer, i.e., within each repeating unit CR¹R²C(=O)O, be H or halogen or any aliphatic or aromatic compound. R¹ and R² are preferably, independently from each other and for each occurrence in the polymer, i.e., within each repeating unit CR¹R²C(O)O, H, halogen, substituted or unsubstituted alkyl, substituted or unsubstituted cycloalkyl, substituted or unsubstituted cycloalkylene alkyl, or substituted or unsubstituted aryl. Examples of a poly(α-hydroxy acid) are poly(lactic acid), PLA, poly(glycolic acid), PGA, or poly(lactic-co-glycolic acid), PLGA. α denotes the alpha C atom. n is, for example, an integer of 2 to 5000. The terms "poly(α-hydroxy acid)", PHA, or "poly(α-hydroxy acid) plastic" also encompass any stereoisomeric, e.g., enantiomeric, forms, e.g., polymers being composed of α-hydroxy acid esters having the same or different stereoisomeric configuration.

R¹ and R² preferably are, independently from each other and for each occurrence in the polymer, i.e., within each repeating unit CR¹R²C(O)O, H, halogen, substituted or unsubstituted C₁₋₂₀ alkyl, substituted or unsubstituted C₁₋₂₀ cycloalkyl, substituted or unsubstituted C₁₋₂₀ cycloalkylene alkyl, substituted or unsubstituted C₁₋₂₀ cycloalkylene dialkyl, or substituted or unsubstituted C₄₋₂₀ aryl. Further preferred, R¹ and R² are, independently from each other and for each occurrence in the polymer, i.e., within each repeating unit CR¹R²C(O)O, H, halogen, substituted or unsubstituted C₁₋₁₅ alkyl, substituted or unsubstituted C₁₋₁₅ cycloalkyl, substituted or unsubstituted C₁₋₁₅ cycloalkylene alkyl, or substituted or unsubstituted C₁₋₁₅ cycloalkylene dialkyl. Still further preferred, R¹ and R² are, independently from each other and for each occurrence in the polymer, i.e., within each repeating unit CR¹R²C(O)O, H, halogen, substituted or unsubstituted C₁₋₁₀ alkyl, substituted or unsubstituted C₁₋₁₀ cycloalkyl, substituted or unsubstituted C₁₋₁₀ cycloalkylene alkyl, substituted or unsubstituted C₁₋₁₀ cycloalkylene dialkyl, or substituted or unsubstituted C₄₋₁₀ aryl. As mentioned above, R¹ and R² can each be the same or different in each repeating unit, i.e., can be identical or different in different repeating units of the same polymer. For example, R¹ and R² can be both H (e.g., in case of poly(glycolic acid), or R¹ can be CH₃ and R² be H (e.g., in case of poly(lactic acid), or R¹ and R² can both be alkyl or aryl, or R¹ can be alkyl and R² be aryl, other vice versa.

The term "alpha hydroxy acid" (α-hydroxy acid), AHA, relates to a carboxylic acid substituted with a hydroxyl (-OH) group on the α carbon atom, i.e., the C atom to which the carboxyl group (C(=O)OH) is bound.

The terms "poly(lactic acid)", PLA, "poly(lactide)", "polylactide" or "poly(lactic acid) plastics" refer to a compound of chemical formula (C₃H₄O₂)ₙ (for example CAS Number: 26100-51-6, 26023-30-3, 26161-42-2 and 26680-10-4), according to the following general structure **1a:** 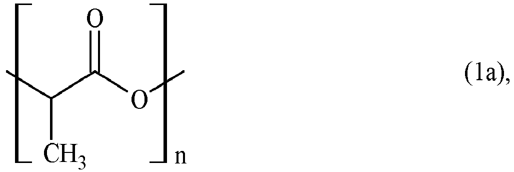 n is the number of lactic acid ester repeating units, which are obtained from lactic acid monomers (for example CAS Number: 10326-41-7, 50-21-5, 79 33-4) and is as defined above. n/2 is the number of lactide monomers, lactide being the lactic acid ester dimer (for example CAS Number: 95-96-5, 4511-42-6, 13076-17-0, 13076-19-2, 26680-10-4) used for the synthesis of the polymer. The terms also encompass any stereoisomeric, e.g., enantiomeric, forms, e.g., polymers being composed of lactic acid ester repeating units having the same or different stereoisomeric configuration. Unless explicitly stated otherwise or the context proves otherwise, the terms "poly(lactic acid" and "poly(lactide)" are used synonymously.

The terms "poly(glycolic acid)", PGA, or "poly(glycolide)" or "polyglycolide" refer to a compound of chemical formula (C₂H₂O₂)ₙ (for example CAS Number: 26124-68-5 and 26009-03-0), according to the following general structure **1b:** 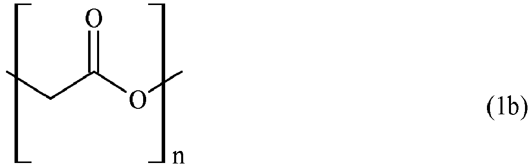 n is the number of glycolic acid ester repeating units, which are obtained from glycolic acid monomers (for example CAS Number: 79-14-1), n/2 is the number of glycolide monomers, glycolide being a glycolic acid ester dimer (for example CAS Number: 502-97-6). n is as defined above. Unless explicitly stated otherwise or the context proves otherwise, the terms "poly(glycolic acid" and "poly(glycolide)" are used synonymously.

The term "poly(lactic-co-glycolic acid)", PLGA, relates to an alternating, random or block copolymer, in particular random or block co-polymers, obtained by polymerization of lactic acid or lactide and glycolic acid or glycolide monomers. Unless explicitly stated otherwise or the context proves otherwise, the terms "poly(lactic-co-glycolic acid)", "poly(lactic acid-co-glycolic acid)" and "poly(lactide-co-glycolide)" are used synonymously.

The terms "a poly(α-hydroxy acid)" or "a poly(α-hydroxy acid) plastic" are not to be construed as being limited to a single type of a poly(α-hydroxy acid) polymer having a specific composition. Rather, the terms include mixtures of different poly(α-hydroxy acid)s, e.g., a mixture of PLA and PGA.

The term "poly(α-hydroxy acid)" encompasses not only any material being a poly(α-hydroxy acid) but also any material comprising a poly(α-hydroxy acid) component besides other components. The terms thus not only encompass co-polymers, as already mentioned above, but also, for example, composite materials comprising a poly(α-hydroxy acid) as, for example, a matrix material, e.g., fiber-reinforced composite materials comprising poly(α-hydroxy acid) plastics as a matrix material and fibers like carbon- or glass-fibers as reinforcing materials.

Terms like "End-of-Life poly(α-hydroxy acid)" or "End-of-Life PAH", e.g., "End-of-Life PLA", refer to poly(α-hydroxy acid) waste material, i.e., a used and/or aged poly(α-hydroxy acid) plastic material, for example used and/or aged PLA material, or a mixture of used and/or aged poly(α-hydroxy acid) materials, that would be subjected to a recycling or waste disposal process. The abbreviation EoL may also be used for the term "End-of-Life".

The term "monomer" in relation to a poly(α-hydroxy acid), e.g., PLA, refers to molecules, which can undergo polymerization, thereby contributing constitutional units to the essential structure of the polymer. For example, lactic acid (2-hydroxypropanoic acid, C₃H₆O₃) is the monomer of poly(lactic acid), PLA, glycolic acid (hydroxyacetic acid) is the monomer making up poly(glycolic acid), PGA. PLGA is composed of the monomers lactic acid and glycolic acid.

The term "repeating unit" in relation to a poly(α-hydroxy acid), e.g., PLA, refers to the respective smallest constitutional unit the repetition of which constitutes the polymer (see, for example, the parts in square brackets of the structures in the formulas presented above). For example, the ester unit C₃H₄O₂ is the repeating unit of poly(lactic acid), PLA, and C₂H₂O₂ is the repeating unit of poly(glycolic acid), PGA. The terms "ester unit", "alpha hydroxy acid ester", "lactic acid ester" or "glycolic acid ester" in relation to a poly(α-hydroxy acid) polymer refer to the fact that the polymer is formally formed by condensation of the respective alpha hydroxy acid(s) under elimination of water, so that the repeating units of the polymer are bound to each other by ester bonds.

A term like "x equivalents of y, based on the repeating unit of the poly(α-hydroxy acid)" means that y is present or added in a molar amount x times the amount of the repeating unit bound in the poly(α-hydroxy acid). An amount of 1.0 equivalents of water, based on the repeating unit of the poly(α-hydroxy acid), thus means that, for an amount of 5 mol poly(α-hydroxy acid) repeating unit, e.g., lactide, 5 mol water are added or present.

A term like "x mol % of y, based on the repeating unit of the poly(α-hydroxy acid)" means that, for each mol of repeating unit x mol % y are used. For example, a presence of 1 mol % of the catalyst zinc acetate (Zn(OAc)₂) means that, in case of a poly(α-hydroxy acid) with 5 mol repeating unit, e.g., lactic acid, 1 % of 5 mol, i.e., 0.05 mol catalyst are present.

The term "contacting a poly(α-hydroxy acid) with water" encompasses any order in which the two components are brought in contact with each other. The poly(α-hydroxy acid) may thus be provided in a first step, with the subsequent addition of water (H₂O) in a second step. Alternatively, water may be provided in a first step, and the poly(α-hydroxy acid) may be subsequently added to the water. It should also be noted here that the term "heating the mixture of the poly(α-hydroxy acid) and water in the presence of a catalyst" is not to be construed as meaning that the catalyst is or is to be added to the mixture of water and poly(α-hydroxy acid). Rather, the three components poly(α-hydroxy acid), water, and catalyst may be added in any order to get the final mixture, including the addition of the catalyst in a first step and the other two components in one or further subsequent steps, or the addition of the catalyst after the previous provision of one of the other components, i.e., water or poly(α-hydroxy acid), and subsequent addition of the third component.

A term, according to which water is "the sole depolymerization reagent and solvent" or "the sole reactant and solvent" in relation to the method of the invention means that no additional solvent like, for example, methanol or ethanol, is used as a depolymerization reagent or solvent in the method. The term thus means that the poly(α-hydroxy acid) is contacted with water as sole depolymerization reagent and solvent, i.e., in the absence of another depolymerization reagent or solvent like, for example, methanol or ethanol. The terms are not to be construed as excluding the presence of a catalyst.

The term "catalyst" refers to any substance, compound or material that modifies and/or increases the rate of a chemical reaction without itself undergoing any permanent chemical change in the reaction. The terms "zinc-based catalyst" or "zinc catalyst" refer to a catalyst containing zinc (Zn).

The term "aliphatic compound" comprises cyclic or acyclic linear (straight chain) or branched, saturated or unsaturated carbon compound residues, other than aromatic residues. The term encompasses "heteroaliphatic compounds", i.e., aliphatic residues in whose carbon skeleton one or more C atoms are replaced by heteroatoms, for example oxygen, sulfur, nitrogen or phosphorus.

The terms "aromatic compound" or "aryl" refer to compounds being or comprising chemical groups with aromaticity, including multi-membered aromatic single ring groups and multicyclic systems with at least one aromatic ring. Examples of aryl groups include benzene, phenyl and naphthalene. The term also includes O-, N-, S- or P-aryl groups, i.e., aryl groups bound to a compound via an oxygen, nitrogen, sulfur or phosphorus atom. The terms encompass, for example, groups of the structure alkyl-aryl-alkyl, e.g., -(CH₂)ₙ-aryl-(CH₂)ₘ-, wherein n and m are, for example, independently from each other 1-10. The terms also encompass heteroaryl groups, i.e., groups having a monocyclic, bicyclic or polycyclic ring, wherein at least one ring is aromatic and contains at least 1 heteroatom, e.g., selected from the group consisting of O, N, P and S.

The term "alkyl" comprises saturated aliphatic (non-aromatic) groups including straight-chain (linear) alkyl groups (e.g., methyl, ethyl, propyl, butyl, pentyl, hexyl, heptyl and octyl), and branched alkyl groups (e.g., isopropyl, tert-butyl, isobutyl). The term also encompasses O, N, S or P alkyl groups (e.g., -O-methyl), i.e., alkyl groups which are bound to a compound via an oxygen, nitrogen, sulfur or phosphorus atom.

The term "cycloalkyl" includes compounds containing an alicyclic group, i.e., a ring-shaped saturated aliphatic (non-aromatic) group, e.g., cyclopropyl, cyclopentyl, cyclohexyl, cycloheptyl, cyclooctyl group.

The term "cycloalkylene alkyl" relates to a compound of the structure -R^{1a}-R^{1b}- or -R^{1b}-R^{1a}-wherein R^{1a} is cycloalkyl and R^{1b} is alkyl, and includes the term "cycloalkylene dialkyl", i.e., a compound of the structure -R^{1b}-R^{1a}-R^{1c}-, wherein R^{1a} is cycloalkyl and R^{1b} and R^{1c} are, independently from each other, alkyl.

The term "Cₙ-Cₘ" or "Cₙ₋ₘ", where n and m are each positive integers and m is greater than n, means a range indicating the number of carbon atoms of a compound or residue. The expression here expressly includes all integer intermediate values between the range boundaries n and m, in each case independently of one another. The expression "C₁₋₁₀" (n = 1, m = 10) therefore means, for example, a compound, group or residue having 1-10, i.e., 1, 2, 3, 4, 5, 6, 7, 8, 9 or 10 carbon atoms. "C₁₋₁₀" therefore also comprises, for example, "C₂₋₆", i.e., 2, 3, 4, 5 or 6 carbon atoms, or "C₁₋₄", i.e., 1, 2, 3 or 4 carbon atoms, or "C₄₋₉", i.e., 4, 5, 6, 7, 8 or 9 carbon atoms. Correspondingly, the term "C₁₋₂₀-alkyl" means, for example, an alkyl group having 1, 2, 3, 4, 5, 6, 7, 8, 9, 10, 11, 12, 13, 14, 15, 16, 17, 18 , 19 or 20 carbon atoms and comprises all combinations of the values of n and m, which lie in the range from n = 1 to m = 20, for example "C₁₋₁₀ alkyl", i.e., an alkyl having 1, 2, 3, 4, 5, 6, 7, 8, 9 or 10 carbon atoms, or "C₅₋₇ alkyl", i.e. an alkyl having 5, 6 or 7 carbon atoms.

The term "halogen" or "halide" refers to chlorine (Cl), fluorine (F), bromine (Br) and iodine (I).

The term "substituted" means that one or more substituents are present, which replace a hydrogen atom on one or more carbon atoms of the hydrocarbon structure. Examples of such substituents are oxo, hydroxyl, phosphate, cyano, azido and amino groups, but also e.g., halogens (e.g., F, Cl, Br), acyl, acyloxy, alkyl, cycloalkyl, heteroalkyl, heterocycloalkyl, aryl and heteroaryl groups. Instead of the term "substituent" also the term "residue" may be used here.

In a preferred embodiment of the method of the invention the poly(α-hydroxy acid) provided in step a) is an End-of-Life poly(α-hydroxy acid).

In a further preferred embodiment of the method of the invention the poly(α-hydroxy acid), preferably End-of-Life poly(α-hydroxy acid), is poly(lactic acid), PLA, poly(glycolic acid), PGA, or poly(lactic-co-glycolic acid), PLGA, preferably PLA.

In a preferred embodiment of the method of the invention the mixture of the poly(α-hydroxy acid) and water is heated, in step b), to a temperature of ≥140 °C in the presence of the catalyst, preferably to a temperature of 140-250 °C, 140-230 °C or 140-200 °C, more preferably to a temperature of ≥160 °C, more preferably to a temperature of 160-250 °C or 160-230 °C, particular preferred to a temperature of 160-200 °C. The heating of the mixture is preferably carried out in a sealed vessel. The reaction can, for example, be carried out in an autoclave or other reactor. The reactor preferably comprises means for heating a sealable compartment being part of the reactor.

The heating is preferably carried out by means of microwave heating. Preferably, the mixture of the poly(α-hydroxy acid), e.g., PLA, and water is thus heated in the presence of the catalyst in a sealed vessel placed in a microwave oven. The reaction can, however, also be carried out in an autoclave or other heatable sealed reactor.

In a preferred embodiment of the method of the invention the heating, for example microwave heating, is carried out over a period of 5-60 min, preferably 10-45 min, 10-40 min, 10-30 min, 10-25 min, 10-20 min, or 15-20 min, particular preferred 20 min. The time periods given refer to the holding time, i.e., the period of time during which the respective heating temperature is maintained.

The catalyst can be any compound that catalyzes the hydrolytic depolymerization of a poly(α-hydroxy acid). Preferably, the catalyst is a zinc-based catalyst, i.e., a compound containing zinc (Zn), preferably having the oxidation state of +2, and being catalytically active in the hydrolytic depolymerization of a poly(α-hydroxy acid). The zinc-based catalyst can, for example, have the structure ZnR¹R², with R¹ and R² being identical or different and being anionic ligands, or have the structure ZnR¹R²Lₙ, with R¹ and R² being as defined above, and L being donor atoms of additional ligands (n = 0-6). In the method of the invention the catalyst is preferably selected from the group consisting of zinc acetate (Zn(OAc)₂), zinc formate (Zn(OOCH)₂), zinc methacrylate (Zn(MA)₂), zinc trifluoromethanosulfonate (Zn(OTf)₂, zinc triflate), zinc halide (e.g., ZnCl₂, ZnF₂, ZnBr₂, ZnI₂), zinc nitrate, zinc phosphate, zinc sulfate, zinc perchlorate, zinc tetrafluoroborate, zinc carbonate, zinc hydroxide, zinc amide, zinc alkoxide, zinc aryloxide, zinc alkoxide aryloxide, zinc alkyl, zinc aryl and zinc oxide (ZnO). The catalyst is preferably Zn(OAc)₂. It is to be noted that the terms and chemical formulas used above for the catalysts encompass any hydrates or solvates of the compounds. For example, the term "zinc acetate" or the formula Zn(OAc)₂ (= Zn(OOCCH₃)₂) comprises the zinc acetate dihydrate (Zn(OOCCH₃)₂·2H₂O). The catalyst is present in the mixture of the poly(α-hydroxy acid) and water before the heating is initiated. The catalyst can be added to the mixture of the poly(α-hydroxy acid) and water, or the mixture of poly(α-hydroxy acid) and water can be added to the catalyst, or the catalyst can be added after addition of any single component of the mixture, before the start of the heating.

Preferably, the catalyst, which preferably is a zinc-based catalyst, and further preferred is a zinc-based catalyst selected from zinc acetate (Zn(OAc)₂), zinc formate (Zn(OOCH)₂), zinc methacrylate (Zn(MA)₂), zinc trifluoromethanesulfonate (Zn(OTf)₂), zinc halide (e.g., ZnCl₂, ZnF₂, ZnBr₂, ZnI₂), zinc nitrate, zinc phosphate, zinc sulfate, zinc perchlorate, zinc tetrafluoroborate, zinc carbonate, zinc hydroxide, zinc amide, zinc alkoxide, zinc aryloxide, zinc alkoxide aryloxide, zinc alkyl, zinc aryl and zinc oxide (ZnO), is present in the mixture in an amount of 0.01-10 mol %, preferably 0.1-10 mol %, 0.1-8.0 mol %, 0.1-5.0 mol %, 0.1-2.0 mol %, 0.1-1.0 mol %, 0.5-1.5 mol % or 0.5-1.0 mol %, further preferred 1.0 mol %, based on the molar amount of repeating unit of the poly(α-hydroxy acid) in the mixture. In a further preferred embodiment of the method of the invention, an amount of 0.1-10 mol %, 0.1-8.0 mol %, 0.1-5.0 mol %, 0.1-2.0 mol %, 0.1-1.0 mol %, 0.5-1.5 mol %, or 0.5-1.0 mol %, preferably 1.0 mol %, zinc acetate (Zn(OAc)₂), based on the molar amount of repeating unit of the poly(α-hydroxy acid), is present in the mixture.

In a preferred embodiment of the method of the invention the poly(α-hydroxy acid) is, in step a), contacted with 1-100 equivalents of water, preferably 1-90, 1-80, or 1-75 equivalents of water, particularly preferred 4-70, 4.5-70, 4.5-60, or 45-50 equivalents of water, based on the repeating unit of the poly(α-hydroxy acid), e.g., PLA.

In a particular preferred embodiment of the method of the invention the poly(α-hydroxy acid) is a poly(lactic acid), PLA, wherein
the PLA is contacted with 1-75 equivalents of water, preferably 4-70, 4.5-70, 4.5-60 or 4.5-50 equivalents of water, based on the repeating unit of the PLA,
and wherein the mixture of the PLA and water is heated in the presence of 0.25-1.5 mol %, preferably 0.5-1.5 mol % or 1.0 mol %, zinc acetate, based on the molar amount of the repeating unit of the PLA in the mixture.

Preferably, in this embodiment, the heating, preferably microwave eating, is carried out for a period of 10-60 min, preferably 10-45 min, 10-40 min or 10-30 min, further preferred 10-25 min, 10-20 min, or 15-20 min, particular preferred 20 min.

In a preferred embodiment of the method of the invention, the heating is carried out under agitation of the mixture to be heated, e.g., agitation by stirring.

In a preferred embodiment of the method of the invention the method further comprises the repolymerization of the poly(α-hydroxy acid). This is particular useful for a recycling process in which a new poly(α-hydroxy acid) is to be synthesized from the products obtained by the depolymerization method of the invention.

In the following, the invention will be described in further detail by way of example only with reference to the accompanying figures.
Figure 1. Schematic diagram of an embodiment of the method of the invention.
Figure 2. Effect of the water loading on the depolymerization of poly(lactic acid) in the presence of 1.0 mol% Zn(OAc)₂ as catalyst, 4.99-49.93 equivalents of water, reaction temperature: 200 °C, reaction time: 20 min microwave (MW) heating.

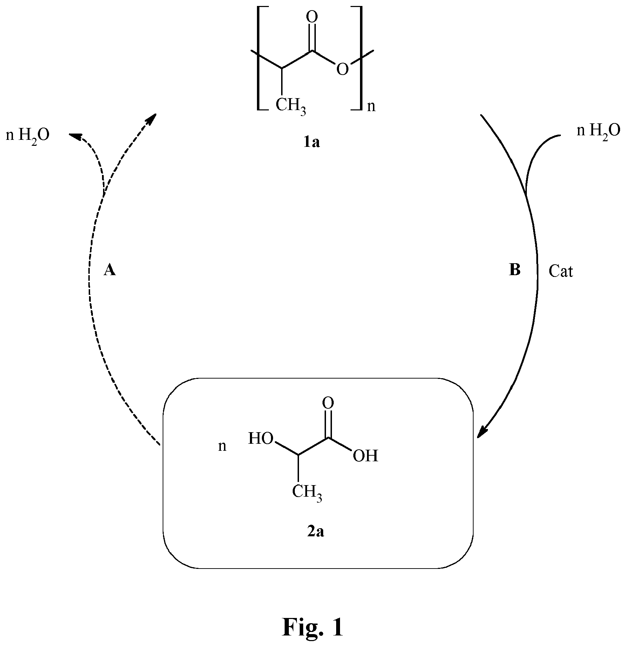

Figure 1 shows a simplified reaction scheme for a recycling process involving an embodiment of the method of the invention. During catalyzed hydrolytic depolymerization (B, solid arrow) of a poly(α-hydroxy acid) **1,** poly(lactic acid), PLA, **1a** being shown here as an example, is converted in the presence of a catalyst (Cat) to the corresponding α-hydroxy acid **2,** here lactic acid **2a.** The latter compound is the industrially relevant starting material for the reverse reaction (polymerization/polycondensation, A, dashed arrow), shown on the left side, to get back to new poly(lactic acid) **1a.** Furthermore, the water (H₂O) used is released again in the polymerization reaction and can be used for the next depolymerization, so that no waste products are produced during chemical recycling.

### Examples

A general scheme for a depolymerization reaction carried out in the method of the invention is presented below:

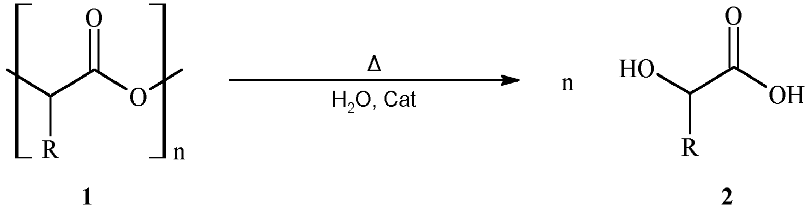

A poly(α-hydroxy acid) **1** is heated in the presence of water (H₂O) and a catalyst to yield the α-hydroxy acid(s) **2** being the monomers of the poly(α-hydroxy acid).

A general scheme of a preferred embodiment of the method of the invention, i.e., a preferred embodiment of the method of the invention for the depolymerization of poly(lactic acid) **1a** to lactic acid **2a,** is shown below:

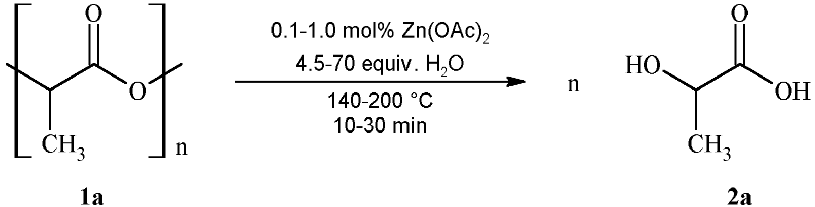

In this embodiment, zinc acetate is used as catalyst.

**General:** All chemicals were used as received without further manipulations. ¹H NMR spectra were recorded on a Fourier 300 MHz (¹H: 300 MHz) or Avance I-400 (¹H: 400 MHz) by Bruker Corporation, using the proton signals of the deuterated solvents as reference. For microwave heating experiments a Microwave Synthesis Reactor Monowave 400 (Anton Paar GmbH, Austria) was used. The reactions can be also performed in an autoclave (Parr Instrument Company, model: 4774).

### General procedure for the depolymerization of polylactide (1a) (optimization of the reaction conditions)

A mixture of polylactide (**1a**, End-of-Life transparent plastic cups; entry #1 of table 3) (200 mg, 2.78 mmol based on the repeating unit), water (3.38 g, 187.7 mmol, 67.5 equiv. based on the repeating unit of **1a**) and catalytic amounts of zinc(II) acetate (0-1.0 mol%, 0-0.0278 mmol based on the repeating unit of **1a**) was placed with a stir bar in a vial. The vial was sealed and subjected to a Microwave Synthesis Reactor Monowave 400. The reaction was performed for 10-30 minutes (hold time) at 140-200 °C and with a stirring of 600 rpm. The reaction temperature was reached within 2 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield **of 2a** was calculated on the basis of the C*H* function **of 2a** and the signal of the C*H₃* function of acetic acid].

Lactic acid (**2a**): ¹H NMR (500 MHz, D2O, 25 °C): δ = 4.38 (pq, 1H, *J* = 7.03 Hz, CH₃C*H*-), 1.42 (d, *J* = 7.03 Hz, 3H, C*H*₃CH-) ppm.

**Table 1. Zn(OAc)₂ catalyzed hydrolytic depolymerization of PLA.**

| 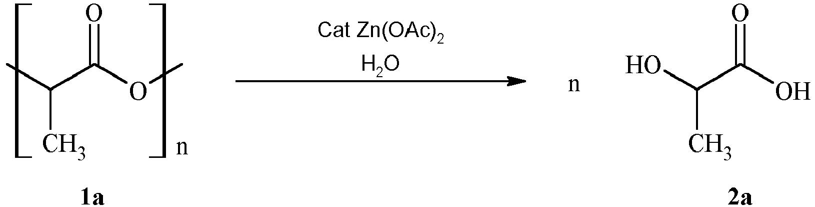 | | | | | |
|---|---|---|---|---|---|
| Entry^{[a]} | Catalyst loading [mol%] | T [°C] | t [min] | Yield [%]^{[b]} | TOF [h⁻¹]^{[c]} |
| 1 | 1.0 | 180 | 30 | 97 | 194 |
| 2 | 1.0 | 160 | 30 | 30 | 61 |
| 3 | 1.0 | 140 | 30 | 3 | 6 |
| 4 | 1.0 | 200 | 30 | 98 | 197 |
| 5 | 1.0 | 200 | 20 | 89 | 249 |
| 6 | 1.0 | 200 | 10 | 55 | 303 |
| 7 | 1.0 | 180 | 10 | 16 | 93 |
| 8 | 0.5 | 200 | 20 | 88 | 489 |
| 9 | 0.25 | 200 | 20 | 87 | 966 |
| 10 | 0.1 | 200 | 20 | 71 | 2136 |
| 11 | - | 200 | 20 | 11 | - |
| 12^{[d]} | 0.25 | 200 | 20 | 82 | 982 |

| | | | | | |
|---|---|---|---|---|---|
| [a] Conditions: **1a** (200.0 mg, 2.78 mmol with respect to the monomer unit), Zn(OAc)₂ (0-1.0 mol%, 0-0.0278 mmol with respect to the monomer unit of **1a**), H₂O (187.7 mmol, 67.5 equiv. with respect to monomer unit of **1a**), temperature: 140-200 °C (microwave heating, reaction vial volume of work: 2.0-6.0 mL), time: 10-30 min. [b] The yield **of 2a** bases on ¹H NMR spectroscopy using acetic acid as standard. [c] The TOF was calculated: (mol product/mol catalyst)*h⁻¹. The TOF was calculated using the yield of **2a** at the designated time. [d] Tap water. | | | | | |

### Procedure for the depolymerization of polylactide (1a) (water loading)

A mixture of polylactide (**1a**, End-of-Life transparent plastic cups; entry #1 of table 3) (200 mg, 2.78 mmol based on the repeating unit), water (13.88-138.8 mmol, 4.99-49.9 equiv. based on the repeating unit of **1a**) and catalytic amounts of zinc(II) acetate (5.1 mg, 1.0 mol%, 0.0278 mmol based on the repeating unit of **1a**) was placed with a stir bar in a vial. The vial was sealed and subjected to a Microwave Synthesis Reactor Monowave 400. The reaction was performed for 20 minutes (hold time) at 200 °C and with a stirring of 600 rpm. The reaction temperature was reached within 2 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield of **2a** was calculated on the basis of the C*H* function of **2a** and the signal of the C*H₃* function of acetic acid].

Lactic acid (**2a**): ¹H NMR (500 MHz, D₂O, 25 °C): δ = 4.38 (pq, ¹H, *J* = 7.03 Hz, CH₃C*H*-), 1.42 (d, *J* = 7.03 Hz, 3H, C*H*₃CH-) ppm.

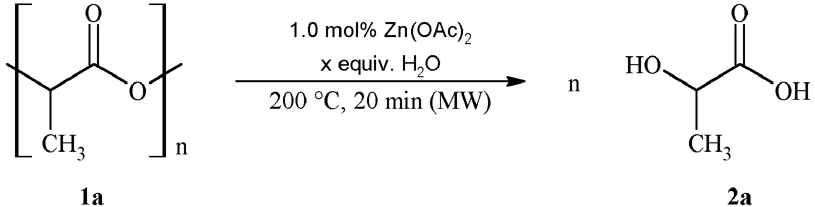

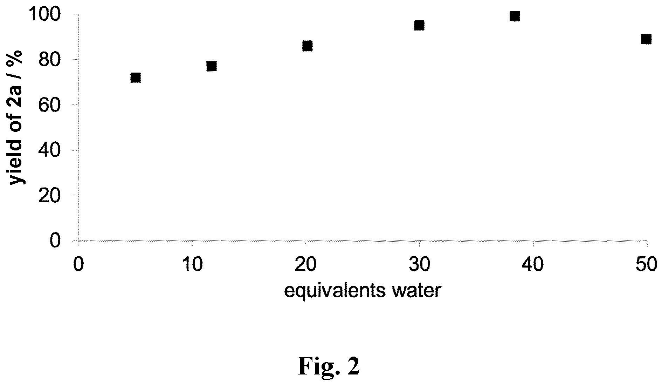

The effect of the water loading on the depolymerization of poly(lactic acid) **1a** is shown in Figure 2. Conditions: **1a** (200.0 mg, 2.78 mmol with respect to the monomer unit), Zn(OAc)₂ (1.0 mol%, 0.0278 mmol with respect to the monomer unit of **1a**), H₂O (4.99-49.93 equiv. with respect to monomer unit of **1a**), temperature: 200 °C (microwave heating, reaction vial volume of work: 0.5-2.0 mL or 2.0-6.0 mL), time: 20 min. The yield of **2a** bases on ¹H NMR spectroscopy using acetic acid as standard.

### Procedure for the depolymerization of polylactide (1a) (zinc salt screening)

A mixture of polylactide (**1a**, End-of-Life transparent plastic cups; entry #1 of table 3) (200 mg, 2.78 mmol based on the repeating unit), water (587 mg, 32.59 mmol, 11.72 equiv. based on the repeating unit of 1a) and catalytic amounts of zinc(II) acetate (5.1 mg, 1.0 mol%, 0.0278 mmol based on the repeating unit of **1a**) was placed with a stir bar in a vial. The vial was sealed and subjected to a Microwave Synthesis Reactor Monowave 400. The reaction was performed for 10 min (hold time) at 200 °C and with a stirring of 600 rpm. The reaction temperature was reached within 2 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield of **2a** was calculated on the basis of the C*H* function of **2a** and the signal of the C*H₃* function of acetic acid].

Lactic acid (**2a**): ¹H NMR (500 MHz, D₂O, 25 °C): δ = 4.38 (pq, 1H, *J* = 7.03 Hz, CH₃C*H*-), 1.42 (d, *J* = 7.03 Hz, 3H, C*H*₃CH-) ppm.

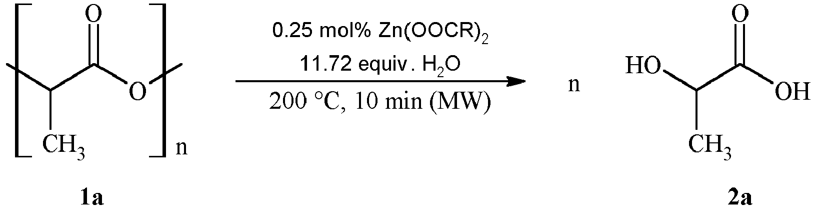

**Table 2. Depolymerization of PLA in the presence of catalytic amounts of zinc(II) carboxylates.**

| Entry^{[a]} | Zinc(II) precatalyst | Yield of **2a** [%]^{[b]} | TOF [h⁻¹]^{[c]} |
|---|---|---|---|
| 1 | Zn(OAc)₂^{[d]} | 26 | 618 |
| 2 | Zn(OOCH)₂^{[d]} | 11 | 255 |
| 3 | ZnEt₂/2 HOOCC(CH₃)₃ | 24 | 582 |
| 4 | ZnEt₂/2 stearic acid | 21 | 609 |
| 5 | ZnEt₂/2 HOOCCH₂Cl | 6 | 145 |
| 6 | ZnEt₂/2 HOOCCCl₃ | 24 | 582 |
| 7 | ZnEt₂/2 HOOCCF₃ | 9 | 218 |
| 8 | ZnEt₂/2 HOOCPh | 11 | 255 |
| 9 | ZnEt₂/2 HOOC(C₆H₄(*p*-F)) | 20 | 473 |
| 10 | ZnEt₂/2 HOOC(C₆H₄(*p*-OCH₃)) | 33 | 800 |
| 11 | Zn(MA)₂^{[d]} | 23 | 545 |

| | | | |
|---|---|---|---|
| [a] Conditions: **1a** (200.0 mg, 2.78 mmol with respect to the monomer unit), Zn(OOCR)₂ (0.25 mol%, 0.007 mmol with respect to the monomer unit of *1a*; the precatalyst was prepared by reaction of ZnEt₂ (1.0 M in hexane) with 2 equiv. of the corresponding acid and afterwards removal of hexane; H₂O (32.59 mmol, 11.72 equiv. with respect to monomer unit of **1a**), temperature: 200 °C (microwave heating, reaction vial volume of work: 0.5-2.0 mL), time: 10 min. [b] The yield of **2a** bases on ¹H NMR spectroscopy using acetic acid as standard. [c] The TOF was calculated: (mol product/mol catalyst)*h⁻¹. The TOF was calculated using the yield of **2a** at the designated time. [d] Commercial source. | | | |

### Procedure for the depolymerization of polylactide (1a) (PLA screening):

A mixture of polylactide (**1a**, 200 mg, 2.78 mmol based on the repeating unit, based on the idealized assumption that the good contains 100% PLA), water (1.01 g, 55.97 mmol, 20.13 equiv. based on the repeating unit of **1a**) and catalytic amounts of zinc(II) acetate (5.1 mg, 1.0 mol%, 0.0278 mmol based on the repeating unit of **1a**) was placed with a stir bar in a vial. The vial was sealed and subjected to a Microwave Synthesis Reactor Monowave 400. The reaction was performed for 20 min (hold time) at 200 °C and with a stirring of 600 rpm. The reaction temperature was reached within 2 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield of **2a** was calculated on the basis of the C*H* function of **2a** and the signal of the C*H₃* function of acetic acid].

Lactic acid (**2a**): ¹H NMR (500 MHz, D₂O, 25 °C): δ = 4.38 (pq, ¹H, *J* = 7.03 Hz, CH₃C*H*-), 1.42 (d, *J* = 7.03 Hz, 3H, C*H*₃CH-) ppm.

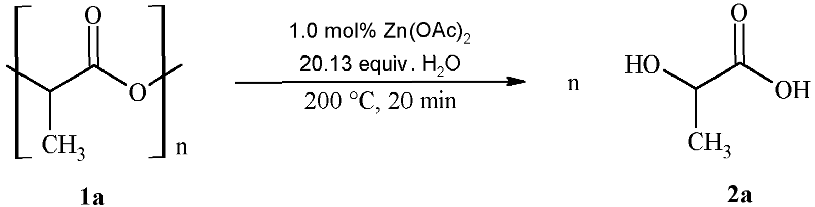

**Table 3. Zn(OAc)₂ catalyzed hydrolytic depolymerization of PLA goods.**

| Entry^{[a]} | Product | Yield of **2a** [%]^{[b]} |
|---|---|---|
| 1 | used transparent cup (1 oz) (**1a.1**) | 86 |
| 2 | used transparent cups (**1a.2**) | 78 |
| 3 | PLA LuminyTM LX175 (colorless) (**1a.3**) | 66 |
| 4 | PLA 6302D NatureWorks Ingeo^{™} (**1a.4**) | 71 |
| 5 | transparent bottle (**1a.5**) | 80 |
| 6 | transparent disposable food box (**1a.6**) | 79 |
| 7 | transparent sushi box cover (**1a.7**) | 86 |
| 8 | transparent PLA foil (**1a.8**) | 99 |
| 9 | off-white spoon (**1a.9**) | 92 |
| 10 | off-white lid for coffee mugs (**1a.10**) | 49 (76)^{[c]} |
| 11 | Yogurt beakers without labels (**1a.11**) | 89 (93)^{[c]} |
| 12 | PLA sandwich panel (**1a.12**) | 74 |
| 13 | PLA based 3D printing filament (black) (**1a.13**) | 66 |
| 14 | sushi box (black base) (**1a.14**) | 71 |

| | | |
|---|---|---|
| [a] Conditions: 1 (200.0 mg, 2.78 mmol with respect to the monomer unit), Zn(OAc)₂ (5.1 mg, 1.0 mol%, 0.0278 mmol with respect to the monomer unit of **1a**), H₂O (20.13 equiv. with respect to monomer unit of **1a**), temperature: 200 °C (microwave heating, reaction vial volume of work: 0.5-2.0 mL). [b] The yield of **2a** bases on ¹H NMR spectroscopy using acetic acid as standard. The amount of substance of **2a** was linked to the amount of substance of PLA. [c] In parenthesis the yield after 40 min is stated. | | |

### Procedure for the depolymerization of polylactide (1a) (scale-up)

A mixture of polylactide (**1a**, End-of-Life transparent plastic cups; entry #1 of table 3) (2.4 g, 33.4 mmol based on the repeating unit), water (12.12 g, 672.3 mmol, 20.13 equiv. based on the repeating unit of **1a**) and catalytic amounts of zinc(II) acetate (61.2 mg, 1.0 mol%, 0.334 mmol based on the repeating unit of **1a**) was placed with a stir bar in a vial. The vial was sealed and subjected to a Microwave Synthesis Reactor Monowave 400. The reaction was performed for 30 min (hold time) at 200 °C and with a stirring of 600 rpm. The reaction temperature was reached within 2 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield of **2a** was calculated on the basis of the C*H* function of **2a** and the signal of the C*H₃* function of acetic acid]. Yield Lactic acid = 94%.

Lactic acid (**2a**): ¹H NMR (500 MHz, D₂O, 25 °C): δ = 4.38 (pq, ¹H, *J* = 7.03 Hz, CH₃C*H*-), 1.42 (d, *J* = 7.03 Hz, 3H, C*H*₃CH-) ppm.

### Procedure for the depolymerization of polylactide (1a) (PLA plus second polymer)

A mixture of polylactide (**1a**, 200 mg, 2.78 mmol based on the repeating unit), polymer B (see table 4 below; 1.39 mmol based on the repeating unit and the repeating unit of **1a**) water (1.01 g, 55.97 mmol, 20.13 equiv. based on the repeating unit of **1**) and catalytic amounts of zinc(II) acetate (5.1 mg, 1.0 mol%, 0.0278 mmol based on the repeating unit of **1a**) was placed with a stir bar in a vial. The vial was sealed and subjected to a Microwave Synthesis Reactor Monowave 400. The reaction was performed for 10 min (hold time) at 200 °C and with a stirring of 600 rpm. The reaction temperature was reached within 2 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield **of 2a** was calculated on the basis of the C*H* function **of 2a** and the signal of the C*H₃* function of acetic acid].

**Table 4. Zn(OAc)₂ catalyzed hydrolytic depolymerization of PLA in the presence of additional polymers.**

| 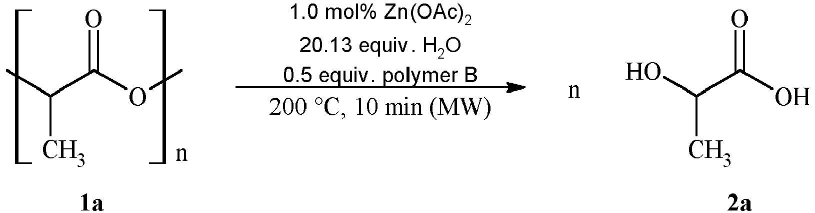 | | | |
|---|---|---|---|
| Entry^{[a]} | Additional polymer B | Yield of **2a** [%]^{[b]} | Yield [%]^{[c]} |
| 1 | - | 37 | - |
| 2 | Poly(ethylene terephthalate) (PET) | 51 | <1 |
| 3 | Poly(ε-caprolactone) (PCL) (Mₙ ∼80,000 g/mol) | 36 | <1 |
| 4 | Nylon 6 | 28 | <1 |
| 5 | Poly(propylene) | 27 | <1 |
| 6 | Poly(ethylene) (PE) (Mₙ ∼1,700 g/mol) | 13 | <1 |
| 7 | Poly(styrene) (PS) (M_{w} ∼35,000 g/mol) | 30 | <1 |
| 8 | Poly(vinyl chloride) (PVC) (Mw ∼48,000 g/mol) | 27 | <1 |

| | | | |
|---|---|---|---|
| [a] Conditions: **1a** (200.0 mg, 2.78 mmol with respect to the monomer unit), polymer B (1.39 mmol with respect to the monomer unit), Zn(OAc)₂ (5.1 mg, 1.0 mol%, 0.0278 mmol with respect to the monomer unit of **1a**), H₂O (1.0 g, 20.13 equiv. with respect to monomer unit of 1a), temperature: 200 °C (microwave heating), temperature: 200 °C (microwave heating), time: 10 min. [b] The yield of **2a** bases on ¹H NMR spectroscopy using acetic acid as standard. [c] The yield was determined by ¹H NMR for depolymerization product(s) of the additional polymer. | | | |

### Procedure for the depolymerization of polylactide (1a) (autoclave scale-up 1)

A mixture of polylactide (**1a**, End-of-Life transparent plastic cups; entry #1 of table 3) (25.0 g, 346.9 mmol based on the repeating unit), water (31.2 g, 1731.2 mmol, 4.99 equiv. based on the repeating unit of **1a**) and catalytic amounts of zinc(II) acetate (636.6 mg, 1.0 mol%, 3.47 mmol based on the repeating unit of **1a**) was placed with a stir bar in an autoclave (volume: 160 mL). The autoclave was sealed and was heated to 160 °C. The reaction was performed for 120 min (hold time) at 160 °C and with a stirring. The reaction temperature was reached within ca. 60 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield of **2a** was calculated on the basis of the CH function of **2a** and the signal of the C*H*₃ function of acetic acid]. Yield Lactic acid = 99%.

Lactic acid (**2a**): ¹H NMR (500 MHz, D₂O, 25 °C): δ = 4.38 (pq, ¹H, *J* = 7.03 Hz, CH₃C*H*-), 1.42 (d, *J* = 7.03 Hz, 3H, C*H*₃CH-) ppm.

### Procedure for the depolymerization of polylactide (1a) (autoclave scale-up 2)

A mixture of polylactide (**1a**, End-of-Life transparent plastic cups; entry #1 of table 3) (25.0 g, 346.9 mmol based on the repeating unit), water (14.1 g, 780.6 mmol, 2.25 equiv. based on the repeating unit of **1a**) and catalytic amounts of zinc(II) acetate (636.6 mg, 1.0 mol%, 3.47 mmol based on the repeating unit of **1a**) was placed with a stir bar in an autoclave (volume: 160 mL). The autoclave was sealed and was heated to 160 °C. The reaction was performed for 120 min (hold time) at 160 °C and with a stirring. The reaction temperature was reached within ca. 60 minutes. Afterwards the vial was cooled to room temperature and acetic acid was added as internal standard. An aliquot of the solution was transferred to an NMR tube and was dissolved in D₂O (0.5 mL). The sample was subjected to ¹H NMR to determine the yield of lactic acid (**2a**) [The yield of **2a** was calculated on the basis of the C*H* function of **2a** and the signal of the C*H*₃ function of acetic acid]. Yield Lactic acid = 99%.

Lactic acid (**2a**): ¹H NMR (500 MHz, D₂O, 25 °C): δ = 4.38 (pq, ¹H, *J* = 7.03 Hz, CH₃C*H*-), 1.42 (d, *J* = 7.03 Hz, 3H, C*H*₃CH-) ppm.

## Claims

1. A method for the depolymerization of a poly(α-hydroxy acid), the method comprising the steps of:
a) contacting the poly(α-hydroxy acid) with water; and
b) heating the mixture of the poly(α-hydroxy acid) and water in the presence of a catalyst suitable for catalyzing the hydrolytic depolymerization of the poly(α-hydroxy acid).

2. The method according to claim 1, wherein the poly(α-hydroxy acid) provided in step a) is an end-of-life poly(α-hydroxy acid).

3. The method according to claim 1 or 2, wherein the poly(α-hydroxy acid) is poly(lactic acid), PLA, poly(glycolic acid), PGA, or poly(lactic-co-glycolic acid), PLGA, preferably PLA.

4. The method according to one of the preceding claims, wherein, in step b), the mixture of the poly(α-hydroxy acid) and water is heated to a temperature of ≥140 °C in the presence of the catalyst, preferably to a temperature of 140-250 °C, 140-230 °C or 140-200 °C, more preferably to a temperature of ≥160 °C, preferably to a temperature of 160-250 °C or 160-230 °C, particular preferred to a temperature of 160-200 °C.

5. The method according to claim 4, wherein the heating is carried out by means of microwave heating.

6. The method according to one of claims 4 or 5, wherein the heating is carried out over a period of 5-60 min, preferably 10-40 min, 10-45 min, 10-30 min, 10-25 min, 10-20 min, or 15-20 min, particular preferred 20 min.

7. The method according to one of the preceding claims, wherein the catalyst is a zinc-based catalyst, preferably selected from the group consisting of zinc acetate, zinc formate, zinc methacrylate, zinc trifluoromethanesulfonate, zinc halide, zinc nitrate, zinc phosphate, zinc sulfate, zinc perchlorate, zinc tetrafluoroborate, zinc carbonate, zinc hydroxide, zinc amide, zinc alkoxide, zinc aryloxide, zinc alkoxide aryloxide, zinc alkyl, zinc aryl and zinc oxide (ZnO), preferably is zinc acetate.

8. The method according to one of the preceding claims, wherein 0.01-10 mol %, preferably 0.1-10 mol %, 0.1-8.0 mol %, 0.1-5.0 mol %, 0.1-2.0 mol %, 0.1-1.0 mol %, 0.25-1.5 mol %, 0.5-1.5 mol %, or 0.5-1.0 mol %, further preferred 1.0 mol %, of the catalyst, preferably zinc-based catalyst, are present in the mixture, based on the molar amount of repeating unit of the α-hydroxy acid in the mixture.

9. The method according to one of the preceding claims, wherein the poly(α-hydroxy acid) is contacted with 1-100 equivalents of water, preferably 1-90, 1-80, or 1-75 equivalents of water, particularly preferred 4-70, 4.5-70, 4.5-60 or 4.5-50 equivalents of water, based on the repeating unit of the poly(α-hydroxy acid).

10. The method according to one of the preceding claims, wherein the poly(α-hydroxy acid) is poly(lactic acid), PLA, and wherein
the PLA is contacted with 4-75 equivalents of water, preferably 4-70, 4.5-70, 4.5-60 or 4.5-50 equivalents of water, based on the repeating unit of the PLA,
and wherein the mixture of the PLA and water is heated in the presence of 0.25-1.5 mol %, preferably 0.5-1.5 mol % or 1.0 mol %, zinc acetate, based on the molar amount of the repeating unit of the PLA in the mixture.

11. The method according to one of the preceding claims, further comprising the repolymerization of the poly(α-hydroxy acid).

12. The method according to one of the preceding claims, wherein water is the sole depolymerization reagent and solvent.
